# EUROPÄISCHE PATENTSCHRIFT

(11) **EP 0 920 844 B1**
(45) Veröffentlichungstag und Bekanntmachung des Hinweises auf die Patenterteilung: **16.04.2008**
(21) Anmeldenummer: 98123220.0
(22) Anmeldetag: 07.12.1998
(51) Int. Cl.: A61F 2/38, A61F 2/28

(54) **Endoprothese zum mindestens teilweisen Ersatz einer Tibia**
Endoprosthesis for the - at least - partial replacement of a tibia
Endoprothèse pour le remplacement au moins partiel du tibia

(30) Priorität: 05.12.1997 DE 19754079
(43) Veröffentlichungstag der Anmeldung: 09.06.1999
(73) Patentinhaber: WALDEMAR LINK GmbH & Co. KG, 22339 Hamburg (DE)
(72) Erfinder: Keller, Arnold, D-23863 Kayhude (DE)
(74) Vertreter: Glawe, Delfs, Moll

(56) Entgegenhaltungen:
- US-A- 5 026 399
- US-A- 5 383 933
- US-A- 5 496 373

## Beschreibung

Die Erfindung besteht in einer Endoprothese zum mindestens teilweisen Ersatz einer Tibia.

Im Tibiabereich können beispielsweise nach umfangreichen Tumorresektionen oder durch das versagen einer Knieendoprothesenverankerung erhebliche Probleme bei der Kraftübertragung vom Kniegelenk zum Sprunggelenk entstehen. Insbesondere ist es denkbar, daß Teile der Tibia resiziert werden müssen, ohne dass die Möglichkeit besteht, eine Verbindung zwischen den Knochenteilen herzustellen, zwischen denen die resizierten Teile entfernt werden mußten. In solchen Fällen mußten häufig Amputationen durchgeführt werden.

Zur Therapie schwerer Defekte am Femur ist aus der US-A-5 026 399 eine stabförmige Prothese bekannt, bei der an beiden Enden Vollgelenksprothesen vorgesehen sind. Nachteilig an dieser Lösung ist, dass nicht nur der geschädigte Knochen ersetzt wird, sondern auch die benachbarten intakten Knochen im ihrem jeweiligen Gelenkbereich zerstört werden, damit die Gelenkprothese an diesen befestigt werden kann.

Aufgabe der vorliegenden Erfindung ist es daher, eine Möglichkeit für eine endoprothetische Versorgung zu schaffen, wenn Teile der Tibia oder die gesamte Tibia entweder entfernt werden mußten oder aber die für eine Kraftübertragung notwendige Festigkeit nicht mehr aufwiesen, und gleichzeitig möglichst viel gesundes Knochen- und Gelenkmaterial zu erhalten.

Diese Aufgabe wird erfindungsgemäß dadurch gelöst, daß an einem sich in Längsrichtung eines Unterschenkels sich erstreckenden Stabteil an dessen einem Kniegelenk zugewandten Oberteil ein Tibiaplateau einer Knieendoprothese befestigt ist und an dessen Unterteil eine sich auf einem natürlichen Sprunggelenk abstützende Ankoppelung vorgesehen ist.

Eine solche Endoprothese kann erfolgreich in denjenigen Fällen eingesetzt werden, wo ein Teil der Tibia entweder völlig fehlt oder so stark geschwächt ist, daß es für die Kraftübertragung nicht mehr geeignet ist. In diesen Fällen wird der Stabteil als Ersatz für die Tibia eingesetzt. Dieser Stabteil kann sich auf seiner gesamten Länge oder nur zum Teil durch die Tibia erstrecken, soweit diese noch erhalten ist. In diesem Falle sind an die den Stabteil bedeckenden Knochen die zur Durchführung von Bewegungen notwendigen Weichteile, wie Bänder, Sehnen und Muskeln angewachsen.

Gemäß einer bevorzugten Ausführungsform der Erfindung ist der Stabteil als ein Rundstab ausgebildet, der an seinem Oberteil eine Ausnehmung aufweist, in die ein im Tibiaplateau befestigter Stift hineinragt, der in der Ausnehmung befestigt ist.

Bei dieser Ausführungsform wird im Regelfall auf bereits bekannte Knieendoprothesen zurückgegriffen. Sie besitzt jedoch an ihrem Tibiateil einen Stift, der abweichend von den bisherigen Ausführungsformen nicht im Tibiaknochen unmittelbar verankert ist, sondern in dem den Tibiaknochen ganz oder zum Teil ersetzenden Stabteil. Im Hinblick darauf, daß bei einer Verankerung des aus Stahl bestehenden Stiftes in einer entsprechenden Ausnehmung des Stabteiles, der auch aus Stahl besteht, relativ hohe Kräfte übertragen werden können, kann mit vergleichsweise kleinen Abmessungen der sich gegenseitig führenden Teile gerechnet werden.

Gemäß einer weiteren bevorzugten Ausführungsform der Erfindung besteht die Ankopplung aus einer gewölbten Platte, die an einem dem Sprunggelenk zugewandten unteren Ende des Stabteiles befestigt ist. Diese Platte kann auf einer vergleichsweise großen Fläche die Kräfte in den Sprunggelenkknochen überleiten. Auf diese Weise ist dafür gesorgt, daß im Bereich der Überleitung der Kräfte vergleichsweise kleine Flächenpressungen auftreten.

Gemäß einer weiteren bevorzugten Ausführungsform der Erfindung schließt die gewölbte Platte nach unten in Richtung auf das Sprunggelenk einen Anpassungsteil ab, der lösbar mit dem Stabteil verbunden ist. Mit Hilfe dieses Anpassungsteiles kann eine genaue Anpassung der gesamten Endoprothese an die jeweils benötigte Länge vorgenommen werden, die zwischen dem Kniegelenk einerseits und dem Sprunggelenk andererseits vorhanden ist.

Gemäß einer weiteren bevorzugten Ausführungsform der Erfindung greift der Anpassungsteil mit seinem dem Sprunggelenk abgewandten oberen Ende in eine am Stabteil befestigte Führungsmuffe ein. Durch diese Art der Befestigung wird sichergestellt, daß der Anpassungsteil zur Übertragung der auftretenden relativ großen Biegemomente gut geführt ist.

Nach einer weiteren bevorzugten Ausführungsform der Erfindung ist der Anpassungsteil in seiner Längsrichtung von einem in der Muffe befestigten Führungsteil geführt, der sich in Längsrichtung parallel zum Anpassungsteil erstreckt. Auf diese Weise wird der Anpassungsteil auf seiner gesamte Länge von dem Führungsteil unterstützt, so daß auch dadurch eine gute Führung des Anpassungsteiles bewirkt wird und eine Lockerung auch bei auftretenden großer Stoßkräfte verhindert wird.

Zu diesem Zwecke sind gemäß einer weiteren bevorzugten Ausführungsform der Erfindung der Führungsteil und der Anpassungsteil im Bereich ihrer sich gegenseitig beaufschlagenden Flächen aufeinander angepaßt. Diese Anpassung verbessert weiterhin die Führung des Anpassungsteiles und verhindert, daß sie sich vom Führungsteil ablösen kann. Die Anpassung kann sowohl form- als auch kraftschlüssig erfolgen.

Zweckmäßigerweise werden weiterhin die Querschnitte des Führungsteiles und des Anpassungsteiles so aufeinander abgestimmt, daß sie gemeinsam etwa dem Querschnitt des Stabteiles entsprechen. Auf diese Weise ist gewährleistet, daß der kraftübertragende Querschnitt der Endoprothese auf deren ganzer Länge gleich groß ist, so daß bruch- oder biegegefährdete Bereiche nicht auftreten können.

Die Führungsmuffe ist zweckmäßigerweise in einem Bereich des Stabteiles befestigt, in dem dieser von Teilen der Tibia knöchern nicht mehr bedeckt ist. Auf diese Weise ist es möglich, sowohl den Führungsteil als auch den Anpassungsteil durch den unteren Teil der Tibia hindurchzuführen. Dadurch wird die Möglichkeit eröffnet, daß Muskeln, Sehnen und Bänder unverändert an den knöchernden Bestandteilen der Tibia angelenkt bleiben.

Gemäß einer weiteren bevorzugten Ausführungsform der Erfindung kann zwischen der gewölbten Platte und dem Sprunggelenkknochen, auf dem sie aufliegt, eine Führung für die Platte vorgesehen sein. Dadurch wird sichergestellt, daß auch bei Stoßbelastungen die Platte von dem Sprunggelenkknochen nicht abruscht. Eine genaue Führung der Endoprothese bezüglich des Sprunggelenkknochens wird erreicht.

Weitere Einzelheiten der Erfindung ergeben sich aus den nachfolgenden ausführlichen Beschreibung und den beigefügten Zeichnungen, in denen bevorzugte Ausführungsformen der Erfindung beispielsweise veranschaulicht sind.

In den Zeichnungen zeigen:

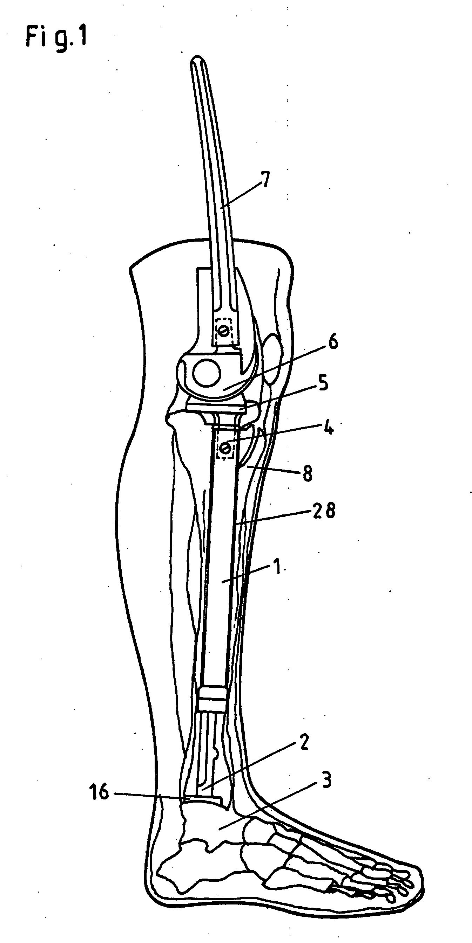

- Figur 1:: Eine schematische Darstellung eines unteren Teiles eines menschlichen Beines mit implantierten Endoprothesen,

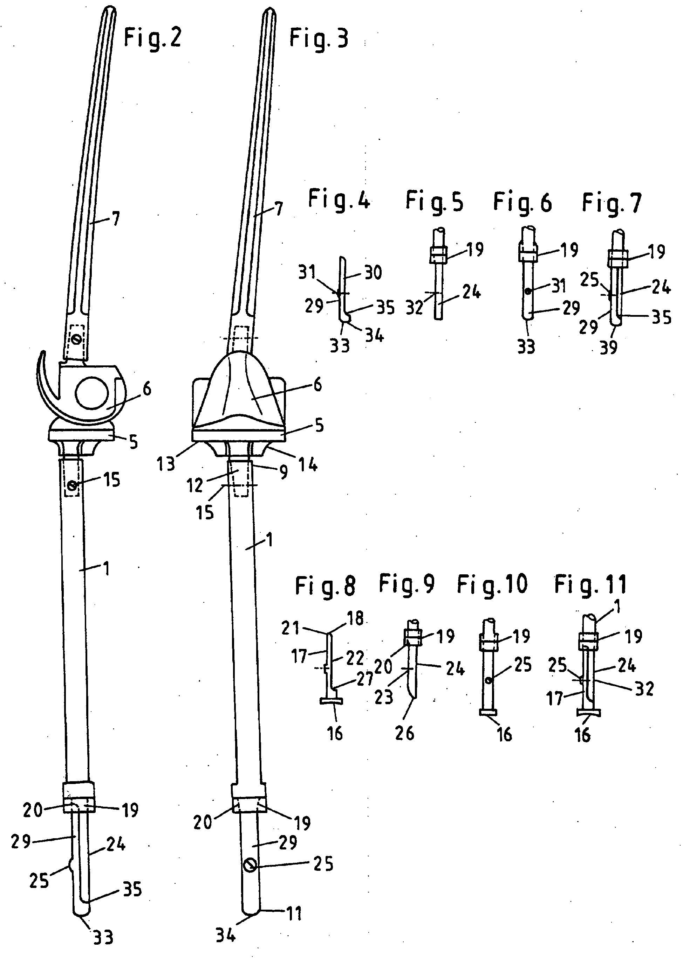

- Figur 2:: eine Seitenansicht eines Endoprothesensystems bestehend aus Femurprothese, Kniegelenkprothese und Tibiaprothese,
- Figur 3:: Vorderansicht eines Endoprothesensystems gemäß Figur 2,
- Figur 4:: Seitenansicht eines Platzhalterteils,
- Figur 5:: Seitenansicht eines Führungsteils,
- Figur 6:: Vorderansicht eines mit einem Platzhalterteil verbundenen Führunsteils,
- Figur 7:: Seitenansicht eines mit einem Platzhalterteil versehenen Führungsteils,
- Figur 8:: Seitenansicht eines Anpassungsteils,
- Figur 9:: Seitenansicht eines Führungsteils,
- Figur 10:: Vorderansicht eines mit einem Anpassungsteil verbundenen Führungsteils und
- Figur 11:: Seitenansicht eines mit einem Führungsteil versehenen Anpassungsteils.

Eine Endoprothese zum mindestens teilweisen Ersatz einer Tibia besteht im wesentlichen aus einem Stabteil 1, einer Ankopplung 2 für ein Sprunggelenk 3 sowie einer Befestigung 4 für ein Tibiaplateau 5 einer Kniegelenkendoprothese 6, an die sich in eine vom Tibiaplateau 5 abgewandte Richtung eine Femurprothese 7 anschließen kann. Der Stabteil 1 erstreckt sich von der für das Tibiaplateau 5 vorgesehenen Befestigung 4 in Richtung auf das Sprunggelenk 3 durch die Tibia 8, die mehr oder minder stark durch Krankheitseinflüsse geschwächt oder sogar völlig fehlen kann.

Der Stabteil 1 kann aus einem Rundstab bestehen, in dessen dem Tibiaplateau zugewandtes oberes Ende 9 eine Ausnehmung 10 eingebracht wird. In dieser Ausnehmung 10, die sich in Richtung auf ein dem Sprunggelenk 3 zugewandtes unteres Ende 11 konisch verjüngen kann, wird ein Stift 12 geführt, der auf einer dem Stabteil 1 zugewandten Unterfläche 13 des Tibiaplateaus 5 befestigt ist. Zur guten Einleitung der vom Tibiaplateau 5 übertragenen Kräfte in den Stabteil 1 kann das Tibiaplateau 5 auf seiner Unterfläche 13 mit überleitenden Flächen 14 versehen sein.

Die Befestigung des Stiftes 12 in der Ausnehmung 10 erfolgt mit Hilfe einer Schraube 15, die sich quer zur Längsrichtung des Stabteiles 1 sowohl durch diesen als auch durch den in die Ausnehmung 10 eingeführten Stift erstreckt. Darüberhinaus ist es möglich, den Stift 12 innerhalb der Ausnehmung 10 formschlüssig zu führen.

Die Ankopplung 2 besteht im wesentlichen aus einer gewölbten Platte 16, die am unteren Ende 11 des Stabteils befestigt ist. Diese gewölbte Platte 16 schließt nach unten in Richtung auf das Sprunggelenk 3 einen Anpassungsteil 17 ab, an dessen unteren Ende die gewölbte Platte 16 befestigt ist.

Der Anpassungsteil 17 wird mit seinem der gewölbten Platte 16 abgewandten oberen Ende 18 in einer Führungsmuffe 19 geführt, in der eine in Richtung auf das Sprunggelenk 3 weisende Einführöffnung 20 vorgesehen ist. In dieser Einführungsöffnung greift das obere Ende 18 des Anpassungsteils mit einer abgerundeten Kante 21 ein. In entsprechender Weise ist die Einführungsöffnung 20 ausgebildet.

Der Anpassungsteil 17 besitzt eine Führungsfläche 22, mit der er nach dem Einführen des oberen Endes 18 in die Einführungsöffnung 20 an einer entsprechenden Fläche 23 eines Führungsteils 24 geführt wird. Dieser Führungsteil 24 ist fest mit der Führungsmuffe 19 verbunden und dient der Führung des Anpassungsteils 17. Dabei können die Flächen 22, 23 formschlüssig ineinander greifen. Sie werden mit Hilfe einer Verbindungsschraube 25 miteinander verbunden, die durch den Anpassungsteil 17 hindurchgreift und mit dem Führungsteil 24 fest verschraubt ist. Auf diese Weise wird eine feste Zuordnung des Anpassungsteils 17 bezüglich des Führungsteils 24 herbeigeführt.

Der Führungsteil 22 und der Anpassungsteil 17 bilden gemeinsam einen Querschnitt, der etwa dem Querschnitt des Stabteils 1 entspricht. Dabei ist der Anpassungsteil 17 im Bereich seiner Führungsfläche 22 etwas stärker ausgebildet als das Führungsstück 24. Dieses ist an seinem dem Sprunggelenk zugewandten unteren Ende 26 abgerundet, so daß es in eine entsprechende Abrundung 27 des Anpassungsteils 17 genau hineinpaßt.

Beim Einbau der Endoprothese in eine Tibia 8 könnte die gewölbte Platte 16 wegen ihrer Ausmaße durch die Tibia 8 nicht hindurchgeführt werden, soweit die Führung des Stabteils 1 innerhalb einer sich durch die Tibia 8 erstreckende Bohrung 28 notwendig ist. Aus diesem Grunde wird der Stabteil 1 nach der Herstellung der Längsbohrung 28 zunächst mit einem Platzhalterteil 29 durch die Längsbohrung 28 durchgeführt. Dieser Platzhalterteil 29 besitzt eine ähnliche Führungsfläche 30 wie der Anpassungsteil 17 mit seiner Führungsfläche 22. Der Platzhalterteil 29 ist auch mit einer Schraubenbohrung 31 versehen, durch die eine Verbindungsschraube hindurchgeführt wird, die mit einem im Führungsteil 24 vorgesehenen Gewinde 32 verschraubt wird. Der wesentliche Unterschied zwischen dem Platzhalterteil 29 und dem Führungsteil 24 besteht jedoch darin, daß der Platzhalterteil 29 an seinem dem Sprunggelenk 3 zugewandten unteren Ende 33 nicht mit einer gewölbten Platte 16 versehen ist, sondern mit einem möglichst abgerundeten Endteil 34. Dieses Endteil 34 kann ohne Schwierigkeit durch die Längsbohrung 28 hindurchgeführt werden, ohne daß Schwierigkeiten im unteren Teil der Tibia 8 entstehen, wo diese besonders schlank ausgebildet ist. Um eine gute Führung des Platzhalterteils 29 am Führungsteil 24 herbeiführen zu können, ist auch beim Platzhalterteil 29 eine der Abrundung 27 entsprechende Auswölbung 35 vorgesehen, die mit dem unteren Ende 26 des Führungsteils 24 korrespondiert und die Führungs des Platzhalterteils 29 unterstützt.

Die Implantation der Endoprothese erfolgt in der Weise, daß zunächst die Längsbohrung 28 vorbereitet wird, soweit noch Bestandteile der Tibia 8 vorhanden sind. Sodann wird durch die Längsbohrung 28 der Stabteil 1 mit dem angeschraubten Platzhalterteil 29 in die Längsbohrung 28 eingeführt. Nach der Einführung wird die Verbindungsschraube 25 gelöst und das Platzhalterteil 29 von dem Führungsteil 24 gelöst. Das Platzhalterteil 29 kann nach unten in Richtung auf das Sprunggelenk 3 aus der Bohrung 28 herausgezogen werden.

Sodann wird an die Stelle des Platzhalterteils 29 das Anpassungsteil 17 so in die Bohrung 28 eingeführt, daß das obere Ende 18 des Anpassungsteils 17 mit seiner abgerundeten Kante in die entsprechende Einführungsöffnung 20 der Führungsmuffe 19 hineinragt. In dieser Stellung werden die beiden Führungsflächen 22, 23 zusammengeführt und das Anpassungsteil 17 mit dem Führungsteil 20 verschraubt. In dieser Stellung wird die gewölbte Platte 16 auf das Sprunggelenk 3 aufgesetzt und gegebenenfalls so ausgerichtet, daß eine Führung zwischen der gewölbten Platte 16 und dem Sprunggelenk 3 zustandekommt. Eine solche Führung kann gegebenenfalls dadurch herbeigeführt werden, daß im Sprunggelenk 3 eine Nut eingebracht wird, die sich in Schwenkrichtung des Fußgelenkes erstreckt. In diese Nut kann ein auf der gewölbten Platte 16 vorgesehener Vorsprung hineinfassen.

Nunmehr wird auf das obere Ende 9 des Stabteils 1 das Tibiaplateau 5 aufgesetzt, indem der Stift 12 in die Ausnehmung 10 eingeführt wird. Schließlich wird der Stift 12 in der Ausnehmung 10 mit Hilfe der Schraube 15 verschraubt.

Auf das in dieser Weise fixierte Tibiaplateau 5 wird der übrige Teil der Kniegelenkendoprothese 6 aufgesetzt. Mit dieser kann gegebenenfalls die Femurprothese 7 verbunden werden.

## Patentansprüche

1. Endoprothese zum mindestens teilweisen Ersatz einer Tibia, **dadurch gekennzeichnet, daß** an einem Stabteil (1) zur Implantierung in Längsrichtung eines Unterschenkels an dessen einem Kniegelenk zugewandten oberen Ende (9) ein Tibiaplateau (5) einer Knieendoprothese (6) befestigt ist und an dessen unteren Ende eine Ankopplung (2) vorgesehen ist, die auf einem natürlichen Sprunggelenk abstützbar ist.

2. Endoprothese nach Anspruch 1, **dadurch gekennzeichnet, daß** der Stabteil (1) als ein Rundstab ausgebildet ist, der an seinem oberen Ende (9) eine Ausnehmung (10) aufweist, in die ein am Tibiaplateau (5) befestigter Stift (12) hineinragt, der in der Ausnehmung (10) befestigt ist.

3. Endoprothese nach Anspruch 2, **dadurch gekennzeichnet, daß** der Stift (12) in der Ausnehmung (10) durch eine quer zur Längsachse des Rundstabes verlaufende Schraube (15) gesichert ist.

4. Endoprothese nach Anspruch 2 oder 3, **dadurch gekennzeichnet, daß** der Stift (12) im Sinne einer konstanten Überleitung der zu übertragenden Kräfte an einer dem Stabteil (1) zugewandten Unterfläche (13) des Tibiaplateaus (5) befestigt ist.

5. Endoprothese nach einem der Ansprüche 1 bis 4, **dadurch gekennzeichnet, daß** die Ankopplung (2) aus einer gewölbten Platte (16) besteht, die an einem dem Sprunggelenk (3) zugewandten unteren Ende des Stabteils (1) befestigt ist.

6. Endoprothese nach Anspruch 5, **dadurch gekennzeichnet, daß** die gewölbte Platte (16) nach unten in Richtung auf das Sprunggelenk (3) einen Anpassungsteil (17) abschließt, der lösbar mit dem Stabteil (1) verbunden ist.

7. Endoprothese nach Anspruch 5 oder 6, **dadurch gekennzeichnet, daß** der Anpassungsteil (17) mit seinem dem Sprunggelenk (3) abgewandten oberen Ende (18) in eine am Stabteil (1) befestigte Führungsmuffe (19) eingreift.

8. Endoprothese nach einem der Ansprüche 5 bis 7, **dadurch gekennzeichnet, daß** der Anpassungsteil (17) in seiner Längsrichtung von einem in der Führungsmuffe (19) befestigten Führungsteil (24) geführt ist, der sich in Längsrichtung parallel zum Anpassungsteil (17) erstreckt.

9. Endoprothese nach Anspruch 8, **dadurch gekennzeichnet, daß** der Führungsteil (24) und der Anpassungsteil (17) im Bereich ihrer sich gegenseitig beaufschlagenden Flächen (22, 23) aufeinander angepaßt sind.

10. Endoprothese nach Anspruch 8 oder 9, **dadurch gekennzeichnet, daß** im Anpassungsteil (17) eine Stützfläche ausgebildet ist, auf der sich eine entsprechende Fläche des Führungsteils (24) abstützt.

11. Endoprothese nach einem der Ansprüche 8 bis 10, **dadurch gekennzeichnet, daß** der Führungsteil (24) und der Anpassungsteil (17) jeweils etwa gleich große Querschnitte aufweisen.

12. Endoprothese nach einem der Ansprüche 8 bis 11, **dadurch gekennzeichnet, daß** die Querschnitte des Führungsteils (24) und des Anpassungsteils (17) gemeinsam etwa dem Querschnitt des Stabteils (1) entsprechen.

13. Endoprothese nach einem der Ansprüche 7 bis 12, **dadurch gekennzeichnet, daß** in der Führungsmuffe (19) auf ihrer dem Sprunggelenk (3) zugewandten Unterseite eine dem Querschnitt des Anpassungsteils (17) angepaßte Einführungsöffnung (20) vorgesehen ist, in der ein dem Sprunggelenk (3) abgewandtes oberes Ende (18) des Anpassungsteils (17) einfaßt.

14. Endoprothese nach einem der Ansprüche 7 bis 13, **dadurch gekennzeichnet, daß** der Führungsteil (24) in der Führungsmuffe (19) befestigt ist.

15. Endoprothese nach einem der Ansprüche 7 bis 14, **dadurch gekennzeichnet, daß** die Führungsmuffe (19) einen größeren Querschnitt als der Stabteil (1) aufweist.

16. Endoprothese nach einem der Ansprüche 7 bis 15, **dadurch gekennzeichnet, daß** die Führungsmuffe (19) oberhalb einer sich in Richtung auf das Sprunggelenk (3) erstreckenden Knochendeckung des Stabteils (1) an diesem befestigt ist.

17. Endoprothese nach einem der Ansprüche 7 bis 16, **dadurch gekennzeichnet, daß** das Führungsteil (24) und das Anpassungsteil (17) gegeneinander verriegelt sind.

18. Endoprothese nach Anspruch 17, **dadurch gekennzeichnet, daß** sich quer zur Richtung des Führungsteils (24) und des Anpassungsteils (17) eine die beiden Teile (24, 17) miteinander verbindende Verbindungsschraube (25) erstreckt.

19. Endoprothese nach Anspruch 18, **dadurch gekennzeichnet, daß** die Verbindungsschraube (25) durch den Anpassungsteil (17) hindurchragt und im Führungsteil (24) verschraubt ist.

20. Endoprothese nach einem der Ansprüche 5 bis 19, **dadurch gekennzeichnet, daß** die gewölbte Platte (16) eine konkave Wölbung aufweist.

21. Endoprothese nach einem der Ansprüche 5 bis 20, **dadurch gekennzeichnet, daß** zwischen der gewölbten Platte (16) und dem Sprunggelenk (3) auf dem sie aufliegt, eine Führung für die Platte (16) vorgesehen ist.

22. Endoprothese nach Anspruch 21, **dadurch gekennzeichnet, daß** die Führung aus einem Führungsvorsprung besteht, der in eine ihm entsprechende Ausnehmung (10) des Sprunggelenkknochens hineingreift.

## Claims

1. Endoprosthesis for at least partial replacement of a tibia, **characterized in that** a tibial plateau (5) of a knee endoprosthesis (6) is secured on the upper end (9), directed towards a knee joint, of a rod part (1) for implantation in the longitudinal direction of a lower leg, and the lower end of said rod part (1) is provided with a coupling part (2) that can be supported on a natural ankle joint.

2. Endoprosthesis according to Claim 1, **characterized in that** the rod part (1) is designed as a round rod which, at its upper end (9), has a recess (10) into which there protrudes a pin (12), which is secured on the tibial plateau (5) and is secured in the recess (10).

3. Endoprosthesis according to Claim 2, **characterized in that** the pin (12) is secured in the recess (10) by a screw (15) extending transverse to the longitudinal axis of the round rod.

4. Endoprosthesis according to Claim 2 or 3, **characterized in that** the pin (12), for the purpose of constant conveying of the forces to be transmitted, is secured on a bottom face (13) of the tibial plateau (5) directed towards the rod part (1).

5. Endoprosthesis according to one of Claims 1 to 4, **characterized in that** the coupling part (2) is composed of a curved plate (16), which is secured on a lower end of the rod part (1) directed towards the ankle joint (3).

6. Endoprosthesis according to Claim 5, **characterized in that** the curved plate (16) forms the downward end, in the direction of the ankle joint (3), of an adapter part (17) connected releasably to the rod part (1).

7. Endoprosthesis according to Claim 5 or 6, **characterized in that** the adapter part (17) engages, via its upper end (18) directed away from the ankle joint (3), in a guide sleeve (19) secured on the rod part (1).

8. Endoprosthesis according to one of Claims 5 to 7, **characterized in that** the adapter part (17) is guided in its longitudinal direction by a guide part (24), which is secured in the guide sleeve (19) and which extends in the longitudinal direction parallel to the adapter part (17).

9. Endoprosthesis according to Claim 8, **characterized in that** the guide part (24) and the adapter part (17) are adapted to each other in the area of their mutually contacting surfaces (22, 23).

10. Endoprosthesis according to Claim 8 or 9, **characterized in that** the adapter part (17) is formed with a support surface on which a corresponding surface of the guide part (24) bears.

11. Endoprosthesis according to one of Claims 8 to 10, **characterized in that** the guide part (24) and the adapter part (17) each have cross sections of approximately the same size.

12. Endoprosthesis according to one of Claims 8 to 11, **characterized in that** the cross sections of the guide part (24) and of the adapter part (17) together correspond approximately to the cross section of the rod part (1).

13. Endoprosthesis according to one of Claims 7 to 12, **characterized in that** the guide sleeve (19), at its lower side directed towards the ankle joint (3), is provided with an insertion opening (20) which is adapted to the cross section of the adapter part (17) and in which an upper end (18) of the adapter part (17) directed away from the ankle joint (3) engages.

14. Endoprosthesis according to one of Claims 7 to 13, **characterized in that** the guide part (24) is secured in the guide sleeve (19).

15. Endoprosthesis according to one of Claims 7 to 14, **characterized in that** the guide sleeve (19) has a larger cross section than the rod part (1).

16. Endoprosthesis according to one of Claims 7 to 15, **characterized in that** the guide sleeve (19) is secured on the rod part (1) above a bone cover thereof extending in the direction of the ankle joint (3).

17. Endoprosthesis according to one of Claims 7 to 16, **characterized in that** the guide part (24) and the adapter part (17) are locked relative to each other.

18. Endoprosthesis according to Claim 17, **characterized in that** a connection screw (25) extending transverse to the direction of the guide part (24) and of the adapter part (17) connects the two parts (24, 17) to each other.

19. Endoprosthesis according to Claim 18, **characterized in that** the connection screw (25) protrudes through the adapter part (17) and is screwed into the guide part (24).

20. Endoprosthesis according to one of Claims 5 to 19, **characterized in that** the curved plate (16) has a concave curvature.

21. Endoprosthesis according to one of Claims 5 to 20, **characterized in that**, between the curved plate (16) and the ankle joint (3) on which it bears, a guide is provided for the plate (16).

22. Endoprosthesis according to Claim 21, **characterized in that** the guide is composed of a guide projection that engages in a corresponding recess (10) of the bone of the ankle joint.

## Revendications

1. Endoprothèse pour le remplacement au moins partiel du tibia, **caractérisée en ce qu'**un plateau de tibia (5) d'une endoprothèse (6) du genou est fixé sur un élément (1) en barre destiné à être implanté dans le sens de la longueur de la jambe et est doté à son extrémité inférieure d'un accouplement (2) qui peut s'appuyer sur l'articulation naturelle de la cheville.

2. Endoprothèse selon la revendication 1, **caractérisée en ce que** l'élément (1) en barre est configuré comme barre ronde qui présente à son extrémité supérieure (9) une découpe (10) dans laquelle pénètre une tige (12) fixée sur le plateau de tibia (5) et fixée dans la découpe (10).

3. Endoprothèse selon la revendication 2, **caractérisée en ce que** la tige (12) est maintenue dans la découpe (10) par une vis (15) qui s'étend transversalement par rapport à l'axe longitudinal de la barre ronde.

4. Endoprothèse selon les revendications 2 ou 3, **caractérisée en ce que** pour permettre un transfert permanent des forces à transmettre, la tige (12) est fixée sur la surface inférieure (13) du plateau de tibia (5) tournée vers l'élément (1) en barre.

5. Endoprothèse selon l'une des revendications 1 à 4, **caractérisée en ce que** l'accouplement (2) est constitué d'une plaque bombée (16) qui est fixée sur l'extrémité inférieure de l'élément (1) en barre tournée vers l'articulation de cheville (3).

6. Endoprothèse selon la revendication 5, **caractérisée en ce que** la plaque bombée (16) ferme un élément d'adaptation (17) vers le bas en direction de l'articulation de cheville (3) et est reliée de manière libérable à l'élément (1) en barre.

7. Endoprothèse selon les revendications 5 ou 6, **caractérisée en ce que** l'élément d'adaptation (17) s'engage par son extrémité supérieure (18) non tournée vers l'articulation de cheville (3) dans un manchon de guidage (19) fixé sur l'élément (1) en barre.

8. Endoprothèse selon les revendications 5 à 7, **caractérisée en ce que** l'élément d'adaptation (17) est guidé dans le sens de sa longueur par un élément de guidage (24) fixé dans le manchon de guidage (19), le sens de la longueur de l'élément de guidage étant parallèle à l'élément d'adaptation (17).

9. Endoprothèse selon la revendication 8, **caractérisée en ce que** l'élément de guidage (24) et l'élément d'adaptation (17) sont adaptés l'un à l'autre dans la zone de leurs surfaces (22, 23) en contact mutuel.

10. Endoprothèse selon les revendications 8 ou 9, **caractérisée en ce qu'**une surface d'appui sur laquelle une surface correspondante de l'élément de guidage (24) s'appuie est formée dans l'élément d'adaptation (17).

11. Endoprothèse selon l'une des revendications 8 à 10, **caractérisée en ce que** l'élément de guidage (24) et l'élément d'adaptation (17) présentent tous deux une section transversale sensiblement de même taille.

12. Endoprothèse selon l'une des revendications 8 à 11, **caractérisée en ce que** la section transversale de l'élément de guidage (24) et celle de l'élément d'adaptation (17) correspondent ensemble sensiblement à la section transversale de l'élément (1) en barre.

13. Endoprothèse selon l'une des revendications 7 à 12, **caractérisée en ce qu'**une ouverture d'introduction (20) adaptée par la section transversale de l'élément d'adaptation (17) est prévue dans le manchon de guidage (19) sur son côté inférieur tourné vers l'articulation de cheville (3), l'extrémité supérieure (18), non tournée vers l'articulation de cheville (3), de l'élément d'adaptation (17) s'engageant dans cette ouverture.

14. Endoprothèse selon l'une des revendications 7 à 13, **caractérisée en ce que** l'élément de guidage (24) est fixé dans le manchon de guidage (19).

15. Endoprothèse selon l'une des revendications 7 à 14, **caractérisée en ce que** le manchon de guidage (19) présente une plus grande section transversale que l'élément (1) en barre.

16. Endoprothèse selon l'une des revendications 7 à 15, **caractérisée en ce que** le manchon de guidage (19) est fixé sur l'élément (1) en barre au-dessus d'un recouvrement de genou, qui s'étend en direction de l'articulation de cheville (3), de l'élément en barre.

17. Endoprothèse selon l'une des revendications 7 à 16, **caractérisée en ce que** l'élément de guidage (24) et l'élément d'adaptation (17) sont verrouillés l'un sur l'autre.

18. Endoprothèse selon la revendication 17, **caractérisée en ce qu'**une vis de liaison (25) qui relie les deux éléments (24, 17) l'un à l'autre s'étend transversalement à la direction de l'élément de guidage (24) et de l'élément d'adaptation (17).

19. Endoprothèse selon la revendication 18, **caractérisée en ce que** la vis de liaison (25) traverse l'élément d'adaptation (17) et est vissée dans l'élément de guidage (24).

20. Endoprothèse selon l'une des revendications 5 à 19, **caractérisée en ce que** la plaque bombée (16) présente une courbure concave.

21. Endoprothèse selon l'une des revendications 5 à 20, **caractérisée en ce qu'**un guide pour la plaque (16) est prévu entre la plaque bombée (16) et l'articulation de cheville (3) sur laquelle elle repose.

22. Endoprothèse selon la revendication 21, **caractérisée en ce que** le guide est constitué d'une saillie de guidage qui s'engage dans une découpe complémentaire (10) de l'os de l'articulation de cheville.
